# EUROPEAN PATENT APPLICATION

(11) **EP 1 669 447 A1**
(43) Date of publication of application: **14.06.2006**
(21) Application number: 04788022.4
(22) Date of filing: 22.09.2004
(51) Int. Cl.: C12N 15/09, C12N 15/12, C12Q 1/68

(54) **SNPs IN 5' REGULATORY REGION OF MDR1 GENE**

(30) Priority: 24.09.2003 JP 2003332584; 18.06.2004 JP 2004181914
(71) Applicant: Kyushu TLO Company, Limited, Fukuoka-shi, Fukuoka 812-8581 (JP)
(72) Inventor: WADA, Morimasa, Kasuga-shi, Fukuoka 8160855 (JP); KUWANO, Michihiko, Fukuoka-shi, Fukuoka 8140013 (JP)
(74) Representative: van Westenbrugge, Andries
(86) International application number: PCT/JP2004/013839
(87) International publication number: WO 2005/028645

(57) **Abstract**

The present invention is to provide a method for determining haplotypes or diplotypes of MDR1 gene targeting the 5' upstream regulatory region of MDR1 gene encoding P-gp, by focusing to ABC transporter, a P-gp, being expressed in the apical membrane side and transporting a wide range of substrates. By detecting a polymorphism at -934 and/or -692, in addition to a position selected from -2903, -2410, -2352, -1910, -1717, and -1325 in a base sequence of the 5' upstream regulatory region of MDR1 gene, haplotypes or diplotypes of the 5'upstream regulatory region of MDR1 gene are determined. The above positions to detect polymorphism are indicated in relation to a first base of ATG start codon which is set to +1. ATG start codon is located in exon 2, and the transcription start site corresponds to -699 in this numbering system.

## Description

### Technical Field

The present invention relates to a method for determining haplotypes or diplotypes of the 5' regulatory region of MDR1 (multidrug resistance 1) gene, particularly to a method for determining haplotypes or diplotypes of the 5' regulatory region of MDR1 gene, by detecting an SNP (single nucleotide polymorphism) at a position selected from -2903, -2410, -2352, -1910, -17170 and -1325, when the position is expressed in relation to a first base of translation start codon ATG which is set to +1, in the base sequence of the 5' regulatory region of MDR1 gene, and the like.

### Background Art

Drugs are detoxified and conjugated in vivo and then exported out of the cells. The activity of the detoxification system affects the pharmacokinetics of drugs. Many recent studies have correlated polymorphisms of detoxification-related genes such as cytochrome P450s and glutathione S-transferases with the efficacy and side effects of drugs (see for example, Roden , D. M. and George , A. L., Jr. The genetic basis of variability in drug responses. Nat Rev Drug Discov, 1: 37-44., 2002; Evans, W. E. and Relling, M. V. Pharmacogenomics: translating functional genomics into rational therapeutics. Science, 286: 487-491., 1999; Gonzalez, F. J., Skoda, R. C., Kimura, S., Umeno, M., Zanger, U. M., Nebert, D. W., Gelboin, H. V., Hardwick, J. P., and Meyer, U. A. Characterization of the common genetic defect in humans deficient in debrisoquine metabolism. Nature, 331: 442-446., 1988). On the other hand, the export of drugs has been shown to involve a group of proteins belonging to ATP Binding Cassette (ABC3) transporters (see for example, Konig, J., Nies, A. T., Cui , Y., Leier, I., and Keppler , D. Conjugate export pumps of the multidrug resistance protein (MRP) family: localization, substrate specificity, and MRP2-mediated drug resistance. Biochim Biophys Acta, 1461: 377-394., 1999. ; Gottesman, M. M., Fojo, T. , and Bates, S. E. Multidrug resistance in cancer: role of ATP-dependent transporters. Nat Rev Cancer, 2: 48-58., 2002.; Borst, P., Evers, R., Kool, M. , and Wijnholds, J. A family of drug transporters: the multidrug resistance-associated proteins. J Natl Cancer Inst, 92: 1295-1302., 2000;Holland, I. B., Cole, S. P. C., Kuchler, K., andHiggins, C. F. ABC proteins, from bacteria to man, p. 423-443. UK: Academic Press, 2003.; Wada, M. , Uchiumi, T. , and Kuwano, M. Canalicular multispecific organic anion transporter, ABCC2 . In: S. Broer and C. A. Wagner (eds.), MEMBRANE TRANSPORT DISEASES -Molecular basis of inherited transport defects-, NY: Kluwer Academic/Plenum Publishers, in press, 2003.;Kuwano, M., Uchiumi, T., Hayakawa, H., Ono, M., Wada, M., Izumi, H., and Kohno, K. The basic and clinical implications of ABC transporters, Y-box-binding protein-1 (YB-1) and angiogenesis-related factors in human malignancies. Cancer Sci, 94: 9-14., 2003.). A member of ABC transporters, P-glycoprotein (P-gp), affects the pharmacokinetics of drugs by limiting the rate at which they are absorbed. Thus, inter-individual variations in the levels of activity and expression of ABC transporters might be a critical factor in the development of pharmacokinetics.

The MDR1 gene is a known gene that encodes a 170-kDa transmembrane protein, P-gp, located at the cytoplasmic surface of the cell, and its base sequence is also knwon. P-gp consists of two membrane-spanning domains and two nucleotide-binding domains. Of the various molecular targets, P-gp expression is responsible for cell resistance to the widest variety of anti-cancer drugs (see for example, Scherf, U., Ross, D. T., Waltham, M., Smith, L. H., Lee, J. K., Tanabe, L., Kohn, K. W., Reinhold, W. C., Myers, T. G., Andrews, D. T., Scudiero, D. A., Eisen, M. B. , Sausville, E. A., Pommier, Y., Botstein, D. , Brown, P.O., and Weinstein, J. N. A gene expression database for the molecular pharmacology of cancer. Nat Genet, 24: 236-244., 2000; Fojo, A. T., Ueda, K., Slamon, D. J., Poplack, D. G., Gottesman, M. M., and Pastan, I. Expression of a multidrug-resistance gene in human tumors and tissues. Proc Natl Acad Sci U S A, 84 : 265-269., 1987). P-gp overexpression plays an important role in the acquisition of drug resistance in various cancer cells. The enhanced expression of the MDR1 gene in malignant cancer cells has been attributed to various mechanisms, including nuclear translocation of YB-1 (see for example, Bargou, R. C., Jurchott, K., Wagener, C., Bergmann, S., Metzner, S., Bommert, K., Mapara, M. Y., Winzer, K. J., Dietel, M., Dorken, B., and Royer, H. D. Nuclear localization and increased levels of transcription factor YB-1 in primary human breast cancers are associated with intrinsic MDR1 gene expression. Nat Med, 3: 447-450., 1997; Ohga, T. , Uchiumi, T. , Makino , Y., Koike , K. , Wada, M. , Kuwano, M. , andKohno, K. Direct involvement of the Y-box binding protein YB-1 in genotoxic stress-induced activation of the human multidrug resistance 1 gene. J Biol Chem, 273: 5997-6000., 1998), promoter rearrangement (see for example, Harada , T. , Nagayama, J. , Kohno, K., Mickley, L. A., Fojo, T., Kuwano, M., and Wada, M. Alu-associated interstitial deletions and chromosomal re-arrangement in 2 human multidrug-resistant cell lines. Int J Cancer, 86: 506-511., 2000), and alteration of methylation status at CpG sites on the MDR1 promoter (see for example, Kusaba, H., Nakayama, M., Harada, T., Nomoto, M., Kohno, K., Kuwano, M. , and Wada, M. Association of 5' CpG demethylation and altered chromatin structure in the promoter region with transcriptional activation of the multidrug resistance 1 gene in human cancer cells. Eur J Biochem, 262: 924-932., 1999; Nakayama, M., Wada, M., Harada, T., Nagayama, J., Kusaba, H., Ohshima, K., Kozuru, M. , Komatsu, H. , Ueda, R. , and Kuwano, M. Hypomethylation status of CpG sites at the promoter region and overexpression of the human MDR1 gene in acute myeloid leukemias. Blood, 92: 4296-4307., 1998.; Tada, Y., Wada, M., Kuroiwa, K., Kinugawa, N., Harada, T., Nagayama, J., Nakagawa, M., Naito, S., and Kuwano, M. MDR1 gene overexpression and altered degree of methylation at the promoter region in bladder cancer during chemotherapeutic treatment. Clin Cancer Res, 6: 4618-4627., 2000).

P-gp is expressed in normal cells of various organs, such as intestine, liver, kidney, brain, and placenta (see for example, Thiebaut, F., Tsuruo, T., Hamada, H., Gottesman, M. M., Pastan, I., and Willingham, M. C. Cellular localization of the multidrug-resistance gene product P-glycoprotein in normal human tissues. Proc Natl Acad Sci U S A, 84: 7735-7738., 1987; Sugawara, I., Kataoka, I., Morishita, Y., Hamada, H., Tsuruo, T., Itoyama, S., and Mori, S. Tissue distribution of P-glycoprotein encoded by a multidrug-resistant gene as revealed by a monoclonal antibody, MRK 16. Cancer Res, 48: 1926-1929., 1988.; Cordon-Cardo, C., O'Brien, J. P., Casals, D., Rittman-Grauer, L., Biedler, J. L., Melamed, M. R., and Bertino, J. R. Multidrug-resistance gene (P-glycoprotein) is expressed by endothelial cells at blood-brain barrier sites. Proc Natl Acad Sci U S A, 86: 695-698., 1989). P-gp's wide substrate specificity and apical localization strongly suggest that it plays a critical role in drug disposition in the human body as well as in animal model (see for example, Schinkel, A. H., Mayer , U., Wagenaar, E., Mol , C. A., van Deemter, L . , Smit, J. J., van der Valk, M. A., Voordouw, A. C., Spits, H., van Tellingen, O., Zijlmans, J. M., Fibbe, W. E., and Borst, P. Normal viability and altered pharmacokinetics in mice lacking mdrl-type (drug-transporting) P-glycoproteins. Proc Natl Acad Sci USA, 94: 4028-4033, 1997; Schinkel, A. H. Pharmacological insights from P-glycoprotein knockout mice. Int J Clin Pharmacol Ther, 36: 9-13, 1998; Ambudkar, S. V., Dey, S., Hrycyna, C. A., Ramachandra, M. , Pastan, I., and Gottesman, M. M. Biochemical, cellular, and pharmacological aspects of the multidrug transporter. Annu Rev Pharmacol Toxicol, 39: 361-398., 1999; Watkins, P. B. The barrier function of CYP3A4 and P-glycoprotein in the small bowel. Adv Drug Deliv Rev, 27: 161-170., 1997; Fromm, M. F. The influence of MDR1 polymorphisms on P-glycoprotein expression and function in humans. Adv Drug Deliv Rev, 54: 1295-1310., 2002). In the intestine, P-gp is thought to participate in drug absorption after drug ingestion (see for example, Cordon-Cardo, C., O'Brien, J. P., Boccia, J., Casals, D., Bertino, J. R., and Melamed, M. R. Expression of the multidrug resistance gene product (P-glycoprotein) in human normal and tumor tissues. J Histochem Cytochem, 38: 1277-1287., 1990; Schuetz, E. G., Schinkel, A. H., Relling, M. V., and Schuetz, J. D. P-glycoprotein: a major determinant of rifampicin-inducible expression of cytochrome P4503A in mice and humans. Proc Natl Acad Sci U S A, 93: 4001-4005., 1996; Greiner, B. , Eichelbaum, M. , Fritz, P. , Kreichgauer, H. P. , von Richter, O., Zundler, J., and Kroemer, H. K. The role of intestinal P-glycoprotein in the interaction of digoxin and rifampin. J Clin Invest, 104: 147-153., 1999). At the blood brain barrier, P-gp influences the uptake of substrates into brain (see for example, Thiebaut, F., Tsuruo, T., Hamada, H., Gottesman, M. M., Pastan, I., and Willingham, M. C. Cellular localization of the multidrug-resistance gene product P-glycoprotein in normal human tissues. Proc Natl Acad Sci U S A, 84: 7735-7738., 1987; Schumacher, U. and Mollgard, K. The multidrug-resistance P-glycoprotein (Pgp, MDR1) is an early marker of blood-brain barrier development in the microvessels of the developing human brain. Histochem Cell Biol, 108: 179-182., 1997; Rao, V. V. , Dahlheimer, J. L. , Bardgett, M. E. , Snyder, A. Z., Finch, R. A., Sartorelli, A. C., and Piwnica-Worms, D. Choroid plexus epithelial expression of MDR1 P glycoprotein and multidrug resistance-associated protein contribute to the blood-cerebrospinal-fluid drug-permeability barrier. Proc Natl Acad Sci U S A, 96: 3900-3905., 1999. ; Thiebaut, F., Tsuruo, T., Hamada, H., Gottesman, M. M., Pastan, I., and Willingham, M. C. Immunohistochemical localization in normal tissues of different epitopes in the multidrug transport protein P170: evidence for localization in brain capillaries and crossreactivity of one antibody with a muscle protein. J Histochem Cytochem, 37: 159-164., 1989.). Since MDR1 expression levels vary widely among individuals (see for example, Hinoshita, E. , Uchiumi, T. , Taguchi, K. , Kinukawa, N. , Tsuneyoshi, M., Maehara, Y., Sugimachi, K., and Kuwano, M. Increased expression of an ATP-binding cassette superfamily transporter, multidrug resistance protein 2, in human colorectal carcinomas. Clin Cancer Res, 6: 2401-2407., 2000), these variations may affect the toxicity of drugs and the efficacy of drug treatment from individual to individual through different drug dispositions. Furthermore, these variations may have another clinical relevance as a cancer risk factor, because the present inventors have recently observed the suppression of polyp formation in mdrla, mouse ortholog of MDR1, -disrupted mice (see for example, Mochida, Y., Taguchi, K., Taniguchi, S., Tsuneyoshi, M., Kuwano, H., Tsuzuki, T., Kuwano, M., and Wada, M. The role of P-glycoprotein in intestinal tumorgenesis: disruption of mdrla suppresses polyp formation in Apc Min/+ mice. Carcinogenesis, 24: 1219-1224., 2003.; Yamada, T., Mori, Y., Hayashi, R., Takada, M., Ino, Y., Naishiro, Y., Kondo, T., and Hirohashi, S. Suppression of intestinal polyposis in Mdrl-deficient ApcMin/+ mice. Cancer Res, 63: 895-901., 2003) . On a molecular basis, however, the mechanism underlying the inter-individual variations in the basal expression level is unknown.

Genetic polymorphisms and their association with P-gp level in MDR1 have been reported recently (see for example, Hoffmeyer, S., Burk, O., von Richter, O., Arnold, H. P., Brockmoller, J., Johne, A., Cascorbi, I., Gerloff, T., Roots, I., Eichelbaum, M., and Brinkmann, U. Functional polymorphisms of the human multidrug-resistance gene: multiple sequence variations and correlation of one allele with P-glycoprotein expression and activity in vivo. Proc Natl Acad Sci U S A, 97: 3473-3478., 2000;Ito, S., Ieiri, I., Tanabe, M., Suzuki, A., Higuchi , S., and Otsubo, K. Polymorphism of the ABC transporter genes, MDR1, MRP1 and MRP2/cMOAT, in healthy Japanese subjects. Pharmacogenetics, 11: 175-184., 2001). Hoffmeyer et al. reported 15 single nucleotide polymorphisms (SNPs), including six in the coding region, in healthy Caucasians. They suggested that one of the SNPs, c.3435C<T (exon 26), is correlated with intestinal P-gp expression and uptake of orally administered digoxin, a P-gp substrate. In Japanese subjects, however, c. 3435C>T was reported not to be related to placental expression of P-gp (see for example, Tanabe, M., Ieiri, I., Nagata, N., Inoue, K., Ito, S., Kanamori, Y., Takahashi, M., Kurata, Y., Kigawa, J. , Higuchi, S. , Terakawa, N. , and Otsubo, K. Expression of P-glycoprotein in human placenta: relation to genetic polymorphism of the multidrug resistance (MDR)-1 gene. J Pharmacol Exp Ther, 297: 1137-1143., 2001). Furthermore, in contrast with the report by Hoffmeyer et al., a T allele of c.3435C>T increased the expression level of MDR1 mRNA in duodenal enterocytes of healthy Japanese subjects (see for example, Nakamura, T., Sakaeda, T., Horinouchi, M., Tamura, T., Aoyama, N., Shirakawa, T. , Matsuo, M., Kasuga, M. , and Okumura, K. Effect of the mutation (C3435T) at exon 26 of the MDR1 gene on expression level of MDR1 messenger ribonucleic acid in duodenal enterocytes of healthy Japanese subjects. Clin Pharmacol Ther, 71: 297-303., 2002). c.3435C>T is a silent mutation that does not cause amino acid substitution. Kim et al. (see for example, Kim, R. B., Leake, B. F., Choo, E. F., Dresser, G. K., Kubba , S. V., Schwarz , U. I . , Taylor , A., Xie , H. G. , McKinsey, J. , Zhou, S. , Lan, L. B. , Schuetz, J. D. , Schuetz, E. G., and Wilkinson, G. R. Identification of functionally variant MDR1 alleles among European Americans and African Americans. Clin Pharmacol Ther, 70: 189-199., 2001) reported that P-gp function could be affected by c.2677G>T, A, an SNP at exon 21 producing Ala893Thr and Ala893Ser, respectively, which is partially linked to c.3435C>T. Tanabe et al. (see for example, Tanabe, M., Ieiri, I., Nagata, N., Inoue, K., Ito, S., Kanamori, Y., Takahashi, M., Kurata, Y., Kigawa, J., Higuchi, S., Terakawa, N., and Otsubo, K. Expression of P-glycoprotein in human placenta: relation to genetic polymorphism of the multidrug resistance (MDR)-1 gene. J Pharmacol Exp Ther, 297: 1137-1143., 2001) reported that P-gp expression levels in placenta were affected by c.2677G>T, A. Thus the relationship between the c.3435C>T genotype and biochemical phenotypic P-gp activity appears not to be clearly established as pointed recently (see for example, Sakaeda, T., Nakamura, T., and Okumura, K. Pharmacogenetics of MDR1 and its impact on the pharmacokinetics and pharmacodynamics of drugs. Pharmacogenomics, 4: 397-410, 2003).

Furthermore, the following methods have been proposed: a method for estimating the side effect of immunosuppressive agent such as tacrolimus or cyclosporine, by investigating whether the 2677th base is guanine, or adenine or thymine, in the position of the cDNA sequence-coding region of human MDR1 gene (see for example, Japanese Laid-Open Patent Application No: 2002-223769); a method for diagnosing etiopathogenic by estimating the expression state of downstream genes such as IL-1α gene, PAI-1 gene, MDR1 gene, MMP-3 gene that are affected by the functional change of p53 gene, by investigating if the functional change caused by p53 gene is related to the development of cancer, in a cancer developed by the impairment of p53 gene function (see for example, Japanese Laid-Open Patent Application No: 2002-269).

ABC transporter is a target molecule playing an important role for susceptibility of anticancer agent or internal kinetics, and it is important to reveal the molecular background caused by individual difference of its expression, to perfom personalized treatment. Because many drugs are substrates of P-gp, degree of expression and activity of P-gp can directly affect the therapeutic effectiveness of such agents. Besides pharmacological relevance, inter-individual variety of P-gp SNPs and expression level may have another clinical impact as follows. Recently, the present inventors found the role of P-gp in colorectal carcinogenesis in mice (see for example, Mochida, Y., Taguchi, K., Taniguchi, S., Tsuneyoshi, M., Kuwano, H., Tsuzuki, T. , Kuwano, M. , and Wada, M. The role of P-glycoprotein in intestinal tumorgenesis: disruption of mdrla suppresses polyp formation in Apc Min/+ mice. Carcinogenesis, 24: 1219-1224., 2003.; Yamada, T., Mori, Y., Hayashi, R., Takada, M., Ino, Y., Naishiro, Y., Kondo, T., and Hirohashi, S. Suppression of intestinal polyposis in Mdrl-deficient ApcMin/+ mice. Cancer Res, 63: 895-901., 2003). The present inventors found that DNA damage was significantly increased in mice disrupted in mdr1a, ortholog of human MDR1, compared with wild-type mice. Surprisingly, the present inventors also found that statistically smaller numbers of polyps was generated in mdr1a-disrupted mice compared with wild-type mice under APCMin background. Inter-individual variety of P-gp expression in colon could then be associated with colorectal carcinogenesis in human. The SNPs at the 5' regulatory region of the human MDR1 gene are associated with the expression of MDR1 mRNA and P-gp in colorectal mucosa and liver in the Japanese population. The results would provide a framework for further analysis of the relationship between the SNPs of MDR1 and drug response, and as well as for further assessment of the importance of P-gp in inter-individual variability of drug response and cancer risk. Further, as it is estimated that MDR1 gene is associated with the biologic defense by exclusion of foreign substances, it may be possible to estimate and diagnose the onset of inflammatory intestinal disease, pathology/disease caused by impairment of the biologic defense function by exclusion of foreign substances, pathology/disease depending on cell survival, anti apoptosis function, or the development of pathology/disease due to impairment thereof. Further, by drug development targeting MDR1 beyond the estimation/diagnosis, it is possible to apply the drug to the prevention and treatment of the above disease. The object of the present invention is to focus on P-gp, an ABC transporter expressed on the apical membrane side, and transporting a wide range of substrates, and to provide a method for determining haplotypes or diplotypes of MDR1 gene targeting the 5' regulatory region of MDR1 gene encoding P-gp.

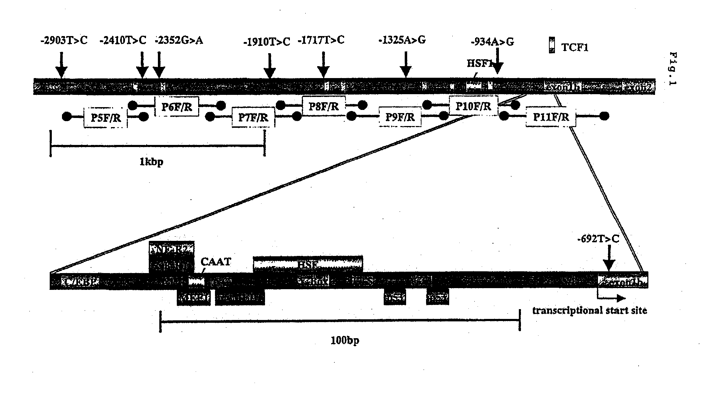

The present inventors analyzed the nucleotide sequence polymorphisms in the 5' regulatory region of the gene spanning 4kb from the transcriptional start site of MDR1 gene in the Japanese population, and identified eight single nucleotide polymorphisms (SNPs) (see Fig. 1) . Of the eight SNPs identified, two (-692T>C and -934A>G) were known, while the six other SNPS (-1325A>G, -1717T>C, -1910T>C, -2352G>A, -2410T>C, and -2903T>C) were not reported and three among these (-1910T>C, -2352G>A, -2410T>C) were in perfect linkage disequilibrium. The present inventors found that: haplotypes or diplotypes are associated with the expression level of MDR1 gene in healthy colon mucous membrane; diplotypes wherein the expression level of MDR1 gene is estimated to be low were not observed in colon cancer patients; and on the contrary, the expression frequency of diplotype wherein the expression level of MDR1 gene is estimated to be high, is higher in the colon cancer patient group than in the control group of healthy subjects; (from statistical analysis, by calculating odds ratio with the use of dyplotype A with high MDR1 , diplotypes B and C with intermediate rate, diplotypes D and E with low MDR1, when diplotype A is set as 1, diplotypes D and E showed 0.524, a half level, and diplotypes B and C showed 0.892, an intermediate rate); the binding ability of a protein binding to the 5' regulatory region of MDR1 gene changes significantly by SNPs; and that 95% or more are converged into 3 haplotypes. Thus, the present inventors obtained knowledge that SNPs of the 5' regulatory region of MDR1 gene are useful as a marker for estimating drug response and oncogenic risk. The present invention has been thus completed.

### Disclosure of the Invention

The present invention relates to a method for determining haplotypes and/or diplotypes of a 5'regulatory region of MDR1 gene, by detecting a polymorphism at a position selected from -2903, -2410, -2352, -1910, -1717 and -1325, when the position is indicated in relation to a first base of translation start codon (ATG) which is set to +1, in a base sequence of a 5'regulatory region of a MDR1 gene ("1"); a method for determining haplotypes and/or diplotypes of a 5'regulatory region of MDR1 gene, by detecting polymorphism at a position selected from -934 and/or -692 position, in addition to the polymorphism at a position selected from -2903, -2410, -2352, -1910, -1717 and -1325, when the position is indicated in relation to a first base of translation start codon (ATG) which is set to +1, in a base sequence of a 5'regulatory region of a MDR1 gene ("2"); the method for determining haplotypes and/or diplotypes of a 5'regulatory region of MDR1 gene according to "1" or "2", comprising the step of investigating whether the base at -2903 is thymine or cytosine ("3"); the method for determining haplotypes and/or diplotypes of a 5'regulatory region of MDR1 gene according to any one of "1" to "3" , comprising the step of investigating whether the base at -2410 is thymine or cytosine ("4"); the method for determining haplotypes and/or diplotypes of a 5'regulatory region of MDR1 gene according to any one of "1" to "4", comprising the step of investigating whether the base at -2352 is guanine or adenine ("5"); the method for determining haplotypes and/or diplotypes of a 5'regulatory region of MDR1 gene according to any one of "1" to "5" , comprising the step of investigating whether the base at -1910 is thymine or cytosine ("6"); the method for determining haplotypes and/or diplotypes of a 5'regulatory region of MDR1 gene according to any one of "1" to "6", comprising the step of investigating whether the base at -1717 is thymine or cytosine ("7"); the method for determining haplotypes and/or diplotypes of a 5'regulatory region of MDR1 gene according to any one of "1" to "7", comprising the step of investigating whether the base at -1325 is guanine or adenine ("8").

Moreover, the present invention relates to a DNA of 5' regulatory region of MDR1 gene, wherein bases at -2410, -2352, -1910 , - -934, -692 are replaced with thymine , adenine, thymine, adenine, thymine, respectively, when the position is indicated in relation to a first base of translation start codon (ATG) which is set to +1, in a base sequence of a 5'regulatory region of MDR1 gene ("9"); a DNA of 5' regulatory region of MDR1 gene, wherein bases at -2410, -2352, -1910, -934, -692 are replaced with cytosine, guanine, cytosine, guanine, cytosine, respectively, when the position is indicated in relation to a first base of translation start codon (ATG) which is set to +1, in a base sequence of a 5'regulatory region of MDR1 gene ("10"); a DNA of 5' regulatory region of MDR1 gene, wherein bases at -2410, -2352, -1910, -934, -692 are replaced with cytosine, adenine, cytosine, guanine, cytosine, respectively, when the position is indicated in relation to a first base of translation start codon (ATG) which is set to +1, in a base sequence of a 5'regulatory region of MDR1 gene ("11"); a DNA of 5' regulatory region of MDR1 gene, wherein bases at -2410, -2352, -1910, -934, -692 are replaced with thymine, adenine, thymine, guanine, thymine, respectively, when the position is indicated in relation to a first base of translation start codon (ATG) which is set to +1, in a base sequence of a 5'regulatory region of MDR1 gene ("12").

Moreover, the present invention relates to a primer set comprising a forward primer that hybridizes with a region upstream of a position for detecting polymorphism, and a reverse primer that hybridizes with a region downstream of a position for detecting polymorphism, which is used for a method for determining haplotypes of a 5' regulatory region of MDR1 gene for detecting a polymorphism at a position selected from -2903, -2410, -2352, -1910, -1717 and -1325, when the position is indicated in relation to a first base of translation start codon (ATG) which is set to +1, in a base sequence of a 5'regulatory region of MDR1 gene ("13"); a method for determining diplotype of 5' regulatory region of MDR1 gene by detecting a polymorphism at -2352, and at a position selected from -2410, -1910 and -692, when the position is indicated in relation to a first base of translation start codon (ATG) which is set to +1, in a base sequence of a 5'regulatory region of MDR1 gene ("14").

Furthermore, the present invention relates to the method for determining diplotype of 5' regulatory region of MDR 1 gene according to "14", wherein gene-typing is performed by TaqMan (registered trademark) method ("15"); a probe and a primer set, used for a method for determining diplotype of 5' regulatory region of MDR1 gene by detecting a polymorphism at -2352, and at a position selected from -2410, -1910 and -692, when the position is indicated in relation to a first base of translation start codon (ATG) which is set to +1, in a base sequence of a 5'regulatory region of MDR1 gene ("16"); the probe and the primer set according to "16" , used for a method for determining diplotype of 5' regulatory region of MDR1 gene by TaqMan (registered trademark) method ("17"); a method for estimating an onset of colon cancer, wherein the method for determining haplotypes and/or diplotypes of 5' regulatory region of MDR1 gene according to any one of "1" to "8", or the method for determining diplotype of 5'regulatory region of MDR1 gene according to "14" or "15" is used ("18"); or a method for developing a drug for controlling MDR1 expression, wherein at least one position selected from -2903, -2410, -2352, -1910, -1717 and -1325, -934, -692 is targeted, when the position is indicated in relation to a first base of translation start codon (ATG) which is set to +1, in a base sequence of a 5'regulatory region of MDR1 gene ("19").

### Brief Description of Drawing

Fig. 1 is a figure showing the positions of SNPs of the 5'regulatory region of MDR1 gene.
Fig. 2 is a figure showing the association between diplotypes at the 5' regulatory region and mRNA level of the MDR1 gene.
   A: Five polymorphisms (-2410, -2352, -1910, -934 and -692) were analyzed at the 5' regulatory region and MDR1 mRNA levels were measured in 72 normal colorectal mucosa by real-time PCR. The 72 samples were divided according to their diplotypes: diplotype A (haplotypes 1/1), diplotype B (haplotypes 1/2), diplotype C (haplotypes 1/3) and diplotype D (haplotypes 2/2). The MDR1 mRNA level was normalized with the GAPDH mRNA level.
   B: Five polymorphisms (-2410, -2352, -1910, -934, -692) at the 5' regulatory region were analyzed and MDR1 mRNA levels were measured in 43 normal liver tissues.

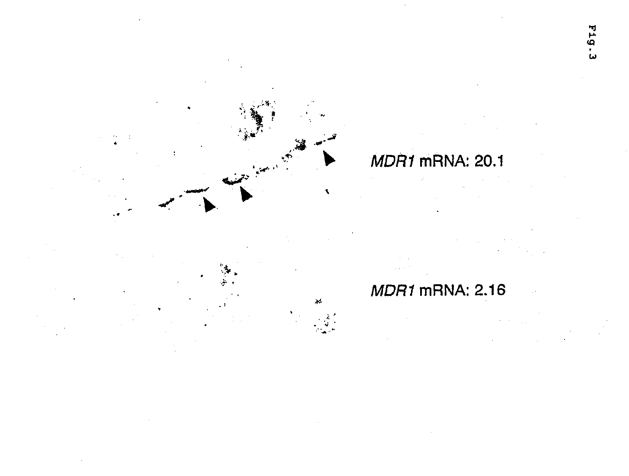

Fig. 3 is a figure showing the results of immunohistochemical staining of P-gp by antibody JSB-1.
   Positive staining for P-gp is observed in the apical membrane tissue of the surface epithelium region. Solid arrowheads indicate P-gp staining by anti-P-gp antibody (JSB-1). The values of MDR1 mRNA level for each sample are shown.

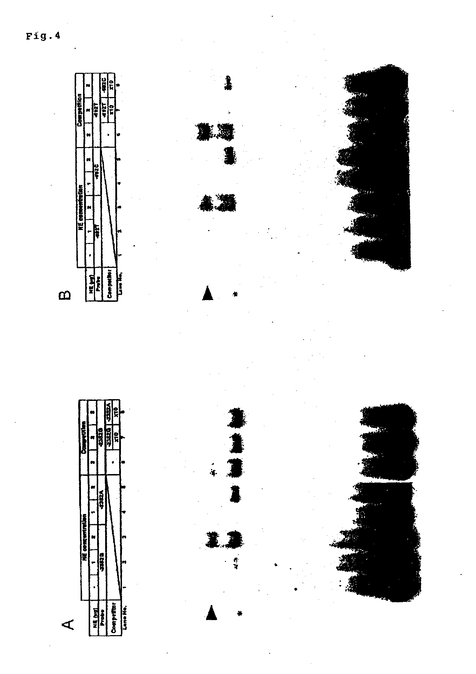

Fig. 4 is a figure showing the detection results of protein binding to the 5' regulatory region of MDR1 by electrophoretic mobility shift assay. The experiments were performed three times each and similar results were obtained. In Fig. 4, NE represents nuclear extract.
   The nuclear extracts (1-2 µg of protein) incubated with ³²P-Iabeled oligonucleotide in binding buffer B (for -2352G>A; panel A) or binding buffer A (for -692T>C; panel B) were resolved by gel electrophoresis. A 10-fold excess of the unlabeled oligonucleotide (-2352G or -2352A) was added for the competition. The solid arrowhead indicates a retarded DNA-protein complex and the asterisk indicates the non-specific binding of nuclear proteins.

### Best Mode of Carrying Out the Invention

As for the method for determining haplotypes and/or diplotypes of a 5'regulatory region of MDR1 gene of the present invention, there is no particular limitation as long as it is a method for detecting a polymorphism at one or more position selected from -2903, -2410, -2352, -1910, -1717 and -1325, when the position is indicated in relation to a first base of translation start codon ATG which is set to +1, in a base sequence of a 5'regulatory region of MDR1 gene. However, a method for detecting a polymorphism at -934 and/or -692, in addition to the above position selected from -2903, -2410, -2352, -1910, -1717 and -1325 is preferable, and particularly, a method for detecting a polymorphism at -2410, -2352, -1910, -934 and -692 is preferable. Herein, the position to detect a polymorphism is indicated by a position in relation to the ATG start codon which is set to +1. ATG start codon is located at exon 2, and the transcription start site corresponds to -699 in this numbering system. Data obtained from gene typing experiments are directly diplotypes, while it is possible to estimate haplotypes reversely from the diplotypes. Every correlational analysis with the expression level or carcinogenesis risk analyses correlation with diplotypes. Further, data obtained from gene typing by TaqMan (registered trademark) method are also diplotypes. Furthermore, complementary sequence (a part of GenBank accession nos. gi/19697556/gb/AC002457.2/, which is a complementary sequence information of the MDR1 gene region) of the 5'regulatory region of MDR1 gene is shown in SEQ ID NO:1. Therefore, it can be seen, for example, that complementary base "T" forming a base pair with the 2410th base "A" of SEQ ID NO:1 is the above -2410 base.

As for a method for detecting a polymorphism at a position selected from -2903, -2410, -2352, -1910, -1717, -1325, -934 and -692 mentioned above (hereinafter sometimes referred to as "predetermined substitution position"), specific examples include: a method for detecting whether the base at -2903 is thymine or cytosine; whether the base at -2410 is thymine or cytosine; whether the base at -2352 is guanine or adenine; whether the base at -1910 is thymine or cytosine; whether the base at -1717 is thymine or cytosine; whether the base at -1325 is adenine or guanine; whether the base at -934 is adenine or guanine; whether the base at -692 is thymine or cytosine, respectively. In the meantime, in SEQ ID NO:1, a normal complementary base sequence of a base sequence of 5' regulatory region of MDR1 gene wherein bases at -2903, -2410, -2352, -1910, -1717, -1325, -934 and -692 are thymine, thymine, guanine, thymine, thymine, adenine, adenine, and thymine, respectively.

As for a method for detecting a polymorphism at a predetermined substitution position, there is no particular limitation as long as it is a method for detecting SNPs with the use of conventionally known methods including PCR, ligand strings reaction, restriction enzyme digestion method, a direct base sequencing analysis, nuclear acid amplification technique, hybridization method, immunoassay, mass spectrometry, etc. For example, it can be performed by the following methods: as the base sequence of the 5'regulatory region of MDR1 gene is already known (see SEQ ID NO:1), a method for directly sequencing with the use of a primer set(SEQ ID NOs: 2 to 25) comprising a forward primer that hybridizes with a region upstream of a predetermined substitution position (position for detecting polymorphism) shown in Table 1 in the following and a reverse primer that hybridizes with a region downstream of a predetermined position, and amplifying by known nucleic acid amplification methods such as PCR to determine the base sequence of the amplified fragment; a method by using TaqMan (registered trademark) probe; a method for investigating restriction fragment length polymorphism (RFLP) of the amplified fragment; a method such as SSCP (single-strand conformation polymorphism); as well as ASO hybridization, ARMS method, denaturing gradient gel electroporesis, RNaseA digestion method, chemical digestion method, DOL method, invader method, MALDI-TOF/MS method, TDI method, molecular beacon method, dynamic allele specific hybridization method, Padlock probe method, UCAN method, nucleic acid hybridization method by using DNA tip or DNA microarray, or ECA method. As long as the above primers have a size to hybridize specifically to the base sequence of the 5'regulatory sequence of MDR1 gene, the size of the primers is not particularly limited, and those having a size of 15 to 40 bases, preferably about 20 bases are generally used, and there is no particular limitation for the size of an amplification regions. Further, genomic DNA to be the template of PCR, can be prepared by common methods from a sample comprising viable cells or dead cells, such as peripheral blood, hair root, oral mucosa, blood smear preparation, regardless of MDR1 expression.

Determination of haplotypes or diplotypes of the 5'regulatory region of MDR1 gene can be determined by detecting a polymorphism at all of the predetermined substitution positions, but it can be determined by detecting polymorphism at certain predetermined substitution positions. For example, when the frequency of minor allele detect a certain level of polymorphism at at -2410, -2352, -1910, -934 and -692, haplotypes or diplotypes can be aggregated.

As for a DNA of the 5' regulatory region of MDR1 gene of the present invention, when the position is indicated in relation to a first base of translation start codon ATG which is set to +1, in a base sequence of a 5'regulatory region of MDR1 gene, a DNA wherein the base at -2410 is replaced with thymine, the base at -2352 with adenine, the base at -1910 with thymine, the base at -934 position with adenine, the base at -692 with thymine; a DNA wherein the base at -2410 is replaced with cytosine, the base at -2352 with guanine, the base at -1910 with cytosine, the base at -934 with guanine, the base at -692 with cytosine; or a DNA wherein the base at -2410 is replaced with cytosine, the base at -2352 with adenine, the base at -1910 with cytosine, the base at -934 with guanine, the base at -692 with cytosine; or a DNA wherein the base at -2410 is replaced with thymine, the base at -2352 with adenine, the base at -1910 with thymine, the base at -934 with guanine, the base at -692 with thymine, can be exemplified. These DNAs constitute a haplotype of the 5'regulatory region of MDR1 gene, with the DNA wherein the base at -2410 is replaced with thymine, the base at -2352 with guanine, the base at -1910 with thymine, the base at -934 with adenine, and the base at -692 with thymine.

As for a primer set of the present invention, there is no particular limitation as long as it is a primer set used for a method for determining haplotypes of the 5'regulatory region of MDR1 gene by detecting a polymorphism at a position selected from -2903, -2410, -2352, -1910, -1717 and -1325, when the position is indicated in relation to a first base of translation start codon ATG which is set to +1, in a base sequence of a 5'regulatory region of MDR1 gene and that comprises a forward primer that hybridizes with a region upstream of a position detecting polymorphism, and a reverse primer that hybridizes with a region downstream of a position for detecting polymorphism, and that each of these primers has a size to hybridize specifically to the base sequence of the 5' regulatory region of MRD1 gene. Generally, those having a size from 15 to 40 bases, preferably those of about 20 bases, are used. The size of an amplifying region is not particularly limited, and specifically includes, for example, primer sets such as P5F/R that detects a polymorphism at -2410 (SEQ ID NOs: 10 and 11); P6F/R that detects a polymorphism at -2352 (SEQ ID NOs: 12 and 13); P7F/R that detects a polymorphism at -1910 (SEQ ID NOs: 14 and 15); P8F/R that detects polymorphism at -1717 (SEQ ID NOs: 16 and 17); and P9F/R that detects a polymorphism at -1325 (SEQ ID NOs: 18 and 19), shown in Table 1.

Further, as for a method for determining diplotype at the 5' regulatory region of MDR1 gene of the present invention, there is no particular limitation as long as it is a method for detecting polymorphism at -2352, and at a position selected from -2410, -1910 and -692, when the position is indicated in relation to a first base of translation start codon ATG which is set to +1, in a base sequence of a 5'regulatory region of MDR1 gene. As it is shown in Table 3 of Example 12 described in the following, -2410T(C), -1910T(C) and -692T(C) are usually detected together in each clone, due to linkage disequilibrium. However, as -2352G(A) is independent, a method for detecting a polymorphism at -2352 and -2410; a method for detecting a polymorphism at -2352 and -1910; or a method for detecting a polymorphism at -2352 and -692 can be specifically exemplified. For example, it is possible to detect polymorphism at -2352G(A) and -692T(C), and to determine/classify into diplotypes such that (-2352, -692, -2352, -692) is (G,T/G,T); (G,T/A,T); (G,T/G,C); (A,T/A,T); (G,C/G,C). As for the method for determining diplotype of the 5' regulatory region of MDR1 gene, a method performing gene typing by TaqMan method can be advantageously exemplified. As for a probe/primer set used for performing gene typing by TaqMan method, for -2352 typing, a probe for detecting G shown in SEQ ID NO: 61; a probe for detecting A shown in SEQ ID NO: 62, and a primer set shown in SEQ ID NOs: 63 and 64; and for -692 typing, a probe for detecting T shown in SEQ ID N0:69, a probe for detecting C shown in SEQ ID N0:70, a primer set shown in SEQ ID NOs : 71 and 72 can be advantageously exemplified.

As it is describe in the above, since diplotypes, that are expected to have a high expression level of MDR1 gene, appear more frequently in the colon cancer patient group than in the normal healthy control group,(from statistical analysis, by calculating odds ratio with the use of dyplotype A with high MDR1 , diplotypes B and C with intermediate rate, diplotypes D and E with low MDR1, when diplotype A is set as 1, diplotypes D and E showed 0.524, and diplotypes B and C showed 0.892, an intermediate rate), by using a method for determining haplotypes and/or diplotypes of the 5'regulatory region of MDR1 gene of the present invention; or a method for determining diplotype of the 5'regulatory region of MDR1 gene, it is possible to estimate the onset of colon cancer. Further, it is possible to develop agents for controlling MDR1 expression targeting at least one position selected from -2903, -2410, -2352, -1910, -1717 and -1325, -934, -692, when the position is indicated in relation to a first base of translation start codon ATG which is set to +1, in a base sequence of a 5'regulatory region of MDR1 gene.

In the following, the present invention will be explained in detail with reference to the examples, while the technical scope of the present invention will not be limited to these examples.

### Example 1

### (Volunteers and patients)

Blood samples were obtained from 25 healthy Japanese volunteers at Kyushu University. Clinical samples of normal colorectal mucosa were taken from 72 Japanese patients who had undergone surgical resection of the cancer at the Department of Surgery II, Kyushu University Hospital (Fukuoka, Japan), the Coloproctology Center, Takano Hospital (Kumamoto, Japan) or the Department of Surgery I, Gunma University Hospital (Maebashi, Japan) between September 1993 and August 1998. These samples of blood and noncancerous mucosa were obtained under an Institutional Review Board (IRB)-approved protocol, with all subjects providing their informed consent. The samples were frozen in liquid nitrogen and stored at -80°C until RNA and DNA were extracted. None of patients had received chemotherapy before the surgical resection. Clinical samples of normal liver tissues were taken as a non-cancerous tissue surrounding cancer from 43 Japanese patients with hepatocellular carcinoma, who had undergone surgical resection of cancer at the Department of Surgery II, Kyushu University Hospital (Fukuoka, Japan).

### Example 2

### (Isolation of DNA and RNA)

Genomic DNA from the volunteers' blood samples was isolated using the QiaAmp (Qiagen) blood kits, and DNA from tissues of patients was isolated using the Easy DNA Kit (Invitrogen) according to the manufacturer's protocol. RNA was isolated using the RNA extraction reagent TRIzol (Invitrogen Life Technologies) or RNeasy (Qiagen) according to the respective manufacturers' protocols.

### Example 3

### (DNA sequencing and identification of SNPs in MDR1 gene)

Specific oligonucleotide primers for PCR amplification of MDR1 gene fragments were derived from known sequences [GenBank accession nos. : AC002457 for the 5' regulatory region and exons 1-7 and AC005068 for exons 8-28]. The locations of the SNPs in the exons corresponded to positions of the MDR1 cDNA (GenBank accession no. M14758 , codon TTC in exon 10, F335 , is missing in that sequence), which the first base of the ATG start codon was set to +1. The exons were defined by Chen et al. (Chen, C. J., Clark, D., Ueda, K., Pastan, I., Gottesman, M. M., and Roninson, I. B. Genomic organization of the human multidrug resistance (MDR1) gene and origin of P-glycoproteins. J Biol Chem, 265: 506-514., 1990), and the Human Genome Organisation recommended nomenclature (Antonarakis, S. E. Recommendations for a nomenclature system for human gene mutations. Nomenclature Working Group. Hum Mutat, 11: 1-3., 1998) was used for SNP nomenclature. The primers were designed to amplify the regions that include sequences including the SNPs reported previously, or to cover about 4kb of the MDR1 upstream regulatory region as shown in Table 1. The PCR conditions for these primers are available by a request to the present inventors. Sequences of purified PCR fragments were obtained by automated DNA sequencing on ABI3700 (capillary) sequencers by using BigDye Terminator cycle sequencing reactions (Perkin-Elmer).

### Example 4

### (Determination of haplotypes at the 5' regulatory region by PCR amplification and sequencing)

Haplotypes of individuals who were heterozygous at least in one SNP locus were determined by PCR amplification and sequencing, using the forward primer MDR1P5F and the reverse primer MDR1P11R. Nucleotide sequences (SEQ ID NOs: 2 to 25) of the 12 primer sets used for screening SNPs of the 5' regulatory region of MDR1 gene spanning for about 4-kb are shown in Table 1. PCR amplification was performed by using high fidelity DNA polymerase, KOD-Plus (Toyobo), according to the manufacture's protocol. The fragments were inserted into pT7Blue-3 vector (Novagen) and subcloned. At least six colonies were picked up, and plasmids were purified by the Qiagen DNA kit according to the manufacturer's protocol. SNP sites were analyzed by sequencing, and haplotypes were confirmed.

**(Table 1) Sequences of oligonucleotide primers used for direct sequencing**

| Primer pair | Product length (bp) |
|---|---|
| MDR1P1F: TATATGTCTCAGCCTGGGCG | 324 |
| MDR1P1R: TCACAGGAGAGCAGACACGT | |
| MDR1P2F: CTCTTGCTCACTCTAGGGAC | 227 |
| MDR1P2R: CAAATATGATCATGAGCCAC | |
| MDR1P3F: CACATATCATCTGAGAAGCCCA | 233 |
| MDR1P3R: AGGACACACCACTTCACTGC | |
| MDR1P4F: AGGCAGTGAAGTGGTGTGTC | 453 |
| MDR1P4R: ACCTTCATTCAAGCGGTGAT | |
| MDR1P5F: ATGAGAGCGGAGGACAAGAA | 469 |
| MDR1P5R: AACCCTCCCTAAACAGTGCA | |
| MDR1P6F: GAGATCTTTACCTGATGCTCA | 355 |
| MDR1P6R: AGGCTTCTAACAGGCCACTA | |
| MDR1P7F: AACAATGCTGTACACTTGCA | 443 |
| MDR1P7R: CTTGGCCTTACAATACAATG | |
| MDR1P8F: CGACAAAGCAAGACTCCGTC | 438 |
| MDR1P8R: CCTTCCATATTTACTGCCAACA | |
| MDR1P9F: GAATTGTGCAGATTGCACG | 437 |
| MDR1P9R: TCCGACCTCTCCAATTCTGT | |
| MDR1P10F: AGCATGCTGAAGAAAGACCA | 380 |
| MDR1P10R: TCAGCCTCACCACAGATGAC | |
| MDR1P11F: CTCGAGGAATCAGCATTCAG | 472 |
| MDR1P11R: GTCCAGTGCCACTACGGTTT | |
| MDR1P12F: GGGACCAAGTGGGGTTAGAT | 474 |
| MDR1P12R: CTTCTTTGCTCCTCCATTGC | |

The nucleotide sequences of 12 primer pairs used to screen the SNPs at the 5' regulatory region of the MDR1 gene spanning about 4kb.

### Example 5

### (Quantitative reverse transcription (RT)-PCR analysis)

Quantitative RT-PCR was performed by real-time Taqman^{™} technology and Model 7900 Sequence Detectors (Perkin-Elmer) as described previously (Gibson, U. E. , Heid, C. A. , and Williams, P. M. A novel method for real time quantitative RT-PCR. Genome Res, 6: 995-1001., 1996). The sequences of the primer pairs and the probe used in this study were described previously (Hinoshita, E., Uchiumi, T., Taguchi, K., Kinukawa, N., Tsuneyoshi, M., Maehara, Y., Sugimachi, K., and Kuwano, M. Increased expression of an ATP-binding cassette superfamily transporter, multidrug resistance protein 2, in human colorectal carcinomas. Clin Cancer Res, 6: 2401-2407., 2000).

### Example 6

### (Cloning of 5' upstream regulatory region of MDR1 into luciferase reporter gene vectors)

To extract type 3 allele, fragments including -2604 to -570 were amplified from templates corresponding to homozygotes for haplotypes 1 and 2, and to heterozygotes for haplotypes 1 and 3. The forward primer 5'-AAAGCTAGCTGTCAGTGGAGCAAAGAAATG-3' (SEQ ID No: 26) and the reverse primer 5'-AAAGCTAGCCTCGCGCTCCTTGGAA-3' (SEQ ID No: 27), each of which included an NheI site, were used. These amplification products were inserted into the NheI site of a pGL3 Basic vector (Promega) . SNP sites in the constructs were confirmed by sequencing.

### Example 7

### (Cell culture and transient transfection)

Human hepatocarcinoma cell line (HepG2) was used in this study. Cells were grown at 37°C in a humidified atmosphere containing 5% carbon dioxide. A total of 1 µg pGL3-Basic Vector DNA or reporter construct was transfected, and then, 100 ng phRL-TK Vector DNA (Promega) was co-transfected in all wells as a transfection control, by using LIPOFECTAMINE 2000 (Life Technologies) reagent and according to the manufacture's protocol. The plates were incubated at 37°C for 6 hr after adding DNA- LIPOFECTAMINE complex, and the growth medium was then changed. The plates were incubated for further 24 hr prior to luciferase assay. Firefly and renilla luciferase activities were measured in a luminometer using the Dual-Luciferase Reporter Assay System (Promega). Data were normalized for transfection efficiency by the Renilla luciferase activity. In all cases, transfections were carried out in triplicate, with 3 wells of a 24-well plate containing identical transfection reactions.

### Example 8

### (Quantitative immunohistochemistry)

The primary antibodies used were P-gp (JSB-1) (mouse monoclonal, Sanbio). Immunostaining of P-gp was performed as described previously. To assure quantitative detection of P-gp by immunohistochemistry, an additional marker protein that is expressed in enterocytes, villin, was used. For quantification, ImageGauge (Fuji Photo Film Co.) software was used.

### Example 9

### (Electrophoretic mobility shift assay)

The DNA sequences of the sense strand of each oligonucleotide were 5'-AAATGAAAGGTGAGATAAAGCAACAA-3' (-2352G; SEQ ID No: 28), 5'-AAATGAAAGGTGAAATAAAGCAACAA-3' (-2352A; SEQ ID No: 29), 5'-GAGCTCATTCGAGTAGCGGCTCTTCC-3' (-692T; SEQ ID No: 30), and 5'-GAGCTCATTCGAGCAGCGGCTCTTCC-3' (-692C; SEQ ID No: 31). Nuclear extracts (2µg/µl of protein) were prepared from HepG2 cells as described previously. They were then incubated for 30 min at room temperature in a final volume of 10µl of reaction mixture containing 2µl of 5x binding buffer; 5mM DTT; 10ng of poly (dI-dC); and 1x10⁴ cpm of ³²P-labeled oligonucleotide probe in the absence or presence of various competitors. The present inventors tried five different kind of binding buffer, determined the one that generated clearest retarded band and used for further analyses.

The composition of the 5x binding buffers used for the detailed analyses were as follows: Buffer A, 60mM HEPES, 300mM KC1, 20mM MgCl₂, 5mM EDTA, 60% (v/v) glycerol; Buffer B, 50mM Tris-HCl (pH 7.5), 250mM NaCl, 12.5mM CaCl₂, 5mM EDTA, 40% (v/v) glycerol. Next, the samples were electrophoresed on 4% polyacrylamide gel (polyacrylamide/bisacrylamide ratio, 79:1) in a Tris-borate-EDTA buffer (0.089 M Tris, 0.089 M Boric acid and 0.002 M EDTA). The gel was exposed to an imaging plate and analyzed using a Fujix BAS 2000 bioimage analyzer (Fuji Photo Film Co.).

### Example 10

### (Statistical analysis)

Statview 5.0 software (SAS Institute) was used for statistical analysis. Results of MDR1 mRNA levels versus diplotypes were analyzed by the Kruskal-Wallis test. Significance was defined as p<0.05. The correlations between MDR1 mRNA level and P-gp level were determined using Spearman's test. This test is usually used for nonparametric analysis when it is unclear whether or not the variables show normal distribution. Probability values of less than 0.05 were considered significant. The Spearman's coefficient (r) and associated probability (P) were calculated. Unpaired t-tests were performed to compare relative luciferase activities of reporter constructs containing haplotype 1, 2, or 3 at the 5' regulatory region of MDR1 gene in transfection experiments.

### Example 11

### (Identification of six new SNPs at the MDR1 5' upstream regulatory region in Japanese)

To find MDR1 polymorphisms at the 5' regulatory region, genomic DNA isolated from peripheral blood of 25 healthy Japanese volunteers was analyzed. The upstream region, spanning about 4kb from the transcriptional start site, was amplified by PCR and analyzed by direct sequencing. Eight SNPs were identified at the 5' regulatory region, and six of them had not been reported before. The allele frequencies of these SNPs observed in the 50 chromosomes are presented in Table 2. The ATG start codon locates in exon2 and the transcription start site corresponds to -699 from the ATG in the genomic DNA. SNPs -692T>C and -934A>G were identical to the previous reported -129T>C and -41aA>G (Tanabe, M., Ieiri, 1., Nagata, N., Inoue, K., Ito, S., Kanamori, Y., Takahashi, M., Kurata, Y., Kigawa, J., Higuchi, S., Terakawa, N., and Otsubo, K. Expression of P-glycoprotein in human placenta: relation to genetic polymorphism of the multidrug resistance (MDR)-1 gene. J Pharmacol Exp Ther, 297: 1137-1143., 2001), respectively, by this numbering system.

The sequences were inspected for deviations from the original MDR1 sequences (GenBank accession nos.: AC002457; AC005068), which we defined as the major-type. The eight SNPs (SEQ ID Nos: 32 to 47) of the 5' regulatory region and the other six SNPs in exons and introns were analyzed: these six SNPs were previously reported to have allele frequencies of more than 0.05 in Caucasians as well as in Japanese (Hoffmeyer, S., Burk, O., von Richter, O., Arnold, H. P., Brockmoller, J., Johne, A., Cascorbi, I., Gerloff, T., Roots, I., Eichelbaum, M., and Brinkmann, U. Functional polymorphisms of the human multidrug-resistance gene: multiple sequence variations and correlation of one allele with P-glycoprotein expression and activity in vivo. Proc Natl Acad Sci U S A, 97: 3473-3478., 2000; Ito, S., Ieiri, I., Tanabe, M., Suzuki, A., Higuchi, S., and Otsubo, K. Polymorphism of the ABC transporter genes, MDR1, MRP1 and MRP2/cMOAT, in healthy Japanese subjects. Pharmacogenetics, 11: 175-184., 2001; and Tanabe, M., Ieiri, I., Nagata, N., Inoue, K., Ito, S., Kanamori, Y., Takahashi, M., Kurata, Y., Kigawa, J., Higuchi, S., Terakawa, N., and Otsubo, K. Expression of P-glycoprotein in human placenta: relation to genetic polymorphism of the multidrug resistance (MDR)-1 gene. J Pharmacol Exp Ther, 297: 1137-1143., 2001). The allele frequencies of these SNPs obtained from analysis of the present inventors are shown in Table 2. The frequencies in Table 2 were calculated from the results of genomic DNA analysis of peripheral blood of SNPs of the 5' regulatory region, coding region and intron region, obtained from 25 healthy volunteers. The allele frequencies were within the range expected from sample size as those reported before. A strong association between c.3435C>T and c.2677G>T, A was observed as previously reported (Tanabe, M., Ieiri, I., Nagata, N., Inoue, K., Ito, S., Kanamori, Y., Takahashi, M., Kurata, Y. , Kigawa, J., Higuchi, S., Terakawa, N., and Otsubo, K. Expression of P-glycoprotein in human placenta: relation to genetic polymorphism of the multidrug resistance (MDR)-1 gene. J Pharmacol Exp Ther, 297: 1137-1143.,2001; Kim, R. B. , Leake, B. F. , Choo, E. F. , Dresser, G. K., Kubba, S. V., Schwarz, U. I., Taylor, A., Xie, H. G., McKinsey, J., Zhou, S., Lan, L. B., Schuetz, J. D., Schuetz, E. G., and Wilkinson, G. R. Identification of functionally variant MDR1 alleles among European Americans and African Americans. Clin Pharmacol Ther, 70: 189-199., 2001), whereas there was no linkage between the polymorphisms at the 5' regulatory region and those of coding region including c.1236C>T, c.2677G>T, A and c.3435C>T. No association between -692T>C and c.2677G>T, A nor c.3435C>T is consistent with previous reports (Kim, R. B., Leake, B. F., Choo, E. F., Dresser, G. K., Kubba, S. V., Schwarz, U. I., Taylor, A., Xie, H. G., McKinsey, J., Zhou, S., Lan, L. B., Schuetz, J. D., Schuetz, E. G., and Wilkinson, G. R. Identification of functionally variant MDR1 alleles among European Americans and African Americans. Clin Pharmacol Ther, 70: 189-199., 2001;Horinouchi, M., Sakaeda, T., Nakamura, T., Morita, Y., Tamura, T., Aoyama, N. , Kasuga, M. , and Okumura, K. Significant genetic linkage of MDR1 polymorphisms at positions 3435 and 2677: functional relevance to pharmacokinetics of digoxin. Pharm Res, 19: 1581-1585, 2002).

**(Table 2) Frequencies of SNPs in the MDR1 gene in the Japanese population**

| Location^{A} | Nucleic substitution (major/minor) | acid | Amino acid substitution | Allele frequency^{B} (major/minor) |
|---|---|---|---|---|
| 5' regulatory region | | | | |
| -2903T >C | AGAGTATAG | / | | 0.98 / 0.02 |
| -2410T >C | AGAGCATAG | | | 0.90 / 0.10 |
| -2352G >A | AGGGTTTAA | / | | 0.72 / 0.28 |
| -1910T >C | AGGGCTTAA | | | 0.90 / 0.10 |
| -1717 T >C | GTGAGATAA | / | | 0.98 / 0.02 |
| -1325A >G | GTGAAATAA | | | 0.98 / 0.02 |
| -934A >G | ATGGTGTGA | / | | 0.90 / 0.10 |
| -692T >C | ATGGCGTGA | | | 0.90 / 0.10 |
| | ATTATGGCT | / | | |
| | ATTACGGCT | | | |
| | CTGGAAAAA | / | | |
| | CTGGGAAAA | | | |
| | CCCAATGAT | / | | |
| | CCCAGTGAT | | | |
| | CGAGTAGCG | / | | |
| | CGAGCAGCG | | | |

| Coding region | | | | |
|---|---|---|---|---|
| c.1236 C >T (exon 12) | AGGGCCTGA | / | Gly412Gly | 0.66 / 0.34 |
| | AGGGTCTGA | | Ala893Ser | 0.50 / 0.36 / 0.14 |
| c.2677 G >T, A (exon 21) | AGGTGCTGG | / | Ala893Thr | |
| | AGGTTCTGG | | Ile1145Ile | 0.58 / 0.42 |
| | | / | | |
| c.3435C>T (exon 26) | AGGTACTGG | | | |
| | AGATCGTGA | / | | |
| | AGATTGTGA | | | |

| Intronic region | | | | |
|---|---|---|---|---|
| IVS4-25 G >T (intron 4) | AATGGTATG | / | | 0.96 / 0.04 |
| | AATGTTATG | | | 0.52 / 0.48 |
| IVS6+139 C >T (intron 6) | GCAACAATG | / | | 0.64 / 0.36 |
| | GCAATAATG | | | |
| IVS16-76 T >A (intron 16) | TTACTAATT | / | | |
| | TTACAAATT | | | |

| | | | | |
|---|---|---|---|---|
| ^{A}. The locations of the SNPs correspond to positions of the MDR1 cDNA, with the first base of the ATG start codon set to +1. The ATG start codon locates in exon2 and the transcription start site corresponds to -699 in this numbering system. | | | | |
| ^{B}. Frequency was calculated from the results of genomic DNA analysis of the peripheral blood of 25 healthy volunteers for the SNPs at the 5' regulatory region as well as at the coding and intronic regions. | | | | |

### Example 12

### (Determination of haplotypes at 5' regulatory region in Japanese)

To unequivocally determine the frequency of haplotypes at the regulatory region, the 2kb fragment containing these polymorphic sites at the -2410, -2352, -1910, -934, and -692 was amplified by PCR. Of 25 blood samples, analysis of homozygous samples at all these sites was omitted, and heterozygous samples were used at least in one of those sites. After subcloning the amplified fragments into the pT7Blue3 vector, their nucleotide sequences were determined. Since the frequencies of the minor alleles at -2903, -1717 and -1325 were too low (0.02) for statistical analysis, these three alleles from the analysis were omitted. The frequencies were calculated from the genetic type of 25 samples of healthy Japanese volunteers, wherein fragments corresponding to the region were confirmed by PCR amplification and sequencing. As shown in Table 3, -2410T(C), -1910T(C) and -692T(C) were detected together in each clone, but -2352G(A) was independent. The haplotypes were determined as follows: haplotype 1 (-2410T, -2352G, -1910T, -934A, -692T), haplotype 2 (-2410T, -2352A, -1910T, -934A, -692T) and haplotype 3 (-2410C, -2352G, -1910C, -934G, -692C). Three haplotypes (haplotypes 1, 2 and 3) accounted for more than 95% of the population. The promoter haplotypes were not associated with any SNPs examined in coding and intron regions in Japanese.

**(Table 3) Haplotypes at the 5' upstream regulatory region of MDR1**

| haplotype | -2410 | -2352 | -1910 | -934 | -692 | Frequency (%) |
|---|---|---|---|---|---|---|
| 1 | T | G | T | A | T | 64 |
| 2 | T | A | T | A | T | 24 |
| 3 | C | G | C | G | C | 8 |
| 4 | C | A | C | G | C | 2 |
| 5 | T | A | T | G | T | 2 |

Frequency was calculated from the genotyping of 25 samples of healthy Japanese volunteers confirmed by PCR amplification and sequencing of corresponding fragments in the region.

### Example 13

### (Association of the haplotypes at the 5' regulatory region of the MDR1 gene with MDR1 mRNA levels in colon and liver of Japanese)

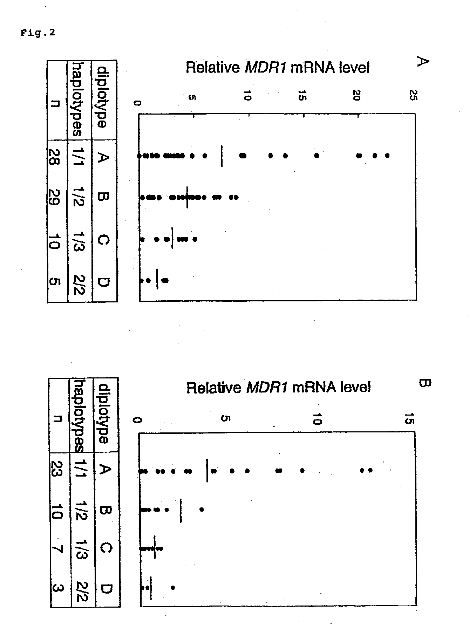

Each of the five polymorphisms (-2410, -2352, -1910, -934, -692) at the 5' regulatory region for any association with MDR1 mRNA levels in 72 normal colorectal mucosa was tested. The 72 samples were divided according to diplotype into four groups: diplotype A (haplotypes 1/1), diplotype B (haplotypes 1/2), diplotype C (haplotypes 1/3) and diplotype D (2/2). Then, it found that the mean MDR1 mRNA level of diplotype A, which had two haplotype 1, was higher than that of diplotype D, which did not have haplotype 1 (p=0.04) (Figure 2A). The mean MDR1 mRNA levels of diplotypes B and C, each of which had one haplotype 1, were intermediate between those of diplotypes A and D. MDR1 mRNA level was normalized with GAPDH mRNA level. When MDR1 mRNA levels in the samples from normal liver were analyzed, results were similar to those from colon (Figure 2B), although the association was statistically much lower compared to that in colon (p=0.2).

Further, each SNP was analyzed for any individual associations with mRNA level. The association between -692T>C and mRNA level in colon was not statistically significant in the present study, although there was a tendency toward lower mRNA levels in T/C heterozygotes than in T/T homozygotes (p=0.2, data not shown). For -2352G>A, A/A homozygotes showed lower mRNA levels than in G/G homozygotes (p=0.07).

Then, a correlation of mRNA expression levels of MDR1 and the expression levels of P-gp was confirmed. Since a sufficient volume of each sample for Western blotting was not available, P-gp levels were measured by immunohistochemistry method using antibody JSB-1. Therefore, the measurements were semi-quantitative rather than quantitative. 14 of the 72 samples were examined and it was found that MDR1 mRNA level showed a significant correlation with P-gp level by Spearman's test (r=0.428, p=0.01). Representative data is presented in Figure 3.

c.3435C>T, which was reported to affect P-gp level in intestine and/or function (Hoffmeyer, S., Burk, O., von Richter, O., Arnold, H. P., Brockmoller, J., Johne, A., Cascorbi, I., Gerloff, T., Roots, I., Eichelbaum, M., and Brinkmann, U. Functional polymorphisms of the human multidrug-resistance gene: multiple sequence variations and correlation of one allele with P-glycoprotein expression and activity in vivo. Proc Natl Acad Sci U S A, 97: 3473-3478., 2000; Nakamura, T., Sakaeda, T., Horinouchi, M., Tamura, T., Aoyama, N., Shirakawa, T., Matsuo, M., Kasuga, M., and Okumura, K. Effect of the mutation (C3435T) at exon 26 of the MDR1 gene on expression level of MDR1 messenger ribonucleic acid in duodenal enterocytes of healthy Japanese subjects. Clin Pharmacol Ther, 71: 297-303., 2002), was not associated with the MDR1 mRNA level in colon and liver in this study (p=0. 7), consistent with the previous report analyzing intestine (Goto, M., Masuda, S., Saito, H., Uemoto, S., Kiuchi, T., Tanaka, K., and Inui, K. C3435T polymorphism in the MDR1 gene affects the enterocyte expression level of CYP3A4 rather than Pgp in recipients of living-donor liver transplantation. Pharmacogenetics, 12: 451-457, 2002) or placenta (Tanabe, M., Ieiri, I., Nagata, N., Inoue, K., Ito, S., Kanamori, Y., Takahashi, M., Kurata, Y., Kigawa, J . , Higuchi , S., Terakawa, N., and Otsubo, K. Expression of P-glycoprotein in human placenta: relation to genetic polymorphism of the multidrug resistance (MDR)-1 gene. J Pharmacol Exp Ther, 297: 1137-1143., 2001). c.2677G>T, A, which was also reported to correlate with the MDR1/P-gp expression level in placenta (Tanabe, M., Ieiri, I., Nagata, N., Inoue, K., Ito, S., Kanamori, Y., Takahashi, M., Kurata, Y., Kigawa, J., Higuchi, S., Terakawa, N., and Otsubo, K. Expression of P-glycoprotein in human placenta: relation to genetic polymorphism of the multidrug resistance (MDR) -1 gene. J Pharmacol Exp Ther, 297: 1137-1143., 2001), was not associated with the MDR1 mRNA level in colon and liver in the present invention (p=0.89), consistent with the results analyzing mRNA level in intestine (the above Pharmacogenetics, 12: 451-457, 2002). None of other SNPs examined in this invention was associated with MDR1 mRNA level in either colorectal epithelium or liver.

### Example 14

### (Comparison analysis of SNP frequencies of MDR1 gene in colon cancer patients and healthy subjects)

To 443 colon cancer patients and 758 of general population of the area in the North region of Kyushu, after explaining them the research purposes and obtaining their informed consent, 10 cc of peripheral blood was collected, and typing was performed by TaqMan method with the use of ABI7900HT (Applied Biosystems) using the extracted genomic DNA as a template. The results are shown in Table 4. As it is clear from Table 4, diplotype E is observed in 9 subjects in control group (healthy subjects), while none was observed in colon cancer patients. Further, by calculating odds ratio with the use of dyplotype A with high MDR1 expression level, diplotypes B and C with intermediate rate, diplotypes D and E with low MDR1, when diplotype A was set to 1, diplotypes D and E showed 0.524 about half rate, and diplotypes B and C showed 0.892, an intermediate rate. From these results, it can be estimated that the risk of colon cancer onset is reduced by half for diplotypes D and E, compared to diplotype A.

**(Table 4)**

| Diplotype | -2352, -692 | Control (%) | Colon cancer patient cases (%) |
|---|---|---|---|
| A | G, T/ G, T | 369 (48.7) | 234 (52.8) |
| B | G, T/ A, T | 266 (35.1) | 153 (34.5) |
| C | G, T/ G, C | 73 (9.6) | 39 (8.8) |
| D | A, T/ A, T | 41 (5.4) | 17 (3.8) |
| E | G, C/ G, C | 9 (1.2) | 0 (0.0) |
| n=1201 | | 758 (100) | 443 (100) |

The above TaqMan method is a method developed by Applied Biosytems, and uses allele-specific probes and region-specific PCR primers having respective SNPs. Then the probes hybridize to the amplification region of PCR, fluorescence generates as the quencher of probe deviates according to the PCR amplification. By measuring the fluorescence, it can be determined whether the allele-specific probe has hybridized or not. Probes for detecting each SNP, and primer sets are as follows:
For -2352 typing
   - Probe for detecting: G:AGGTGAGATAAAGCAA (SEQ ID N0:61)
   - Probe for detecting: A:TGAAAGGTGAAATAAA (SEQ ID N0:62)
   - Primer pair:: Forward: AAGGCCATTCAAAAGGATACATAAAA(SEQ ID NO: 63)
   Reverse: TCTGTTTTCACTTTTGTTTTGCTTTG(SEQ ID NO:64)
For -934 typing
   - Probe for detecting: A:TCCCCAATGATTCAG(SEQ ID NO: 65)
   - Probe for detecting: G:CCCCAGTGATTCAG(SEQ ID NO:66)
   - Primer pair :: Forward: TGTGAACTTTGAAAGACGTGTCTACA(SEQ ID NO: 67)
   Reverse: CAAGTAGAGAAACGCGCATCAG(SEQ ID NO:68)
For -692 typing
   - Probe for detecting: T:TTCGAGTAGCGGCTC(SEQ ID NO:69)
   - Probe for detecting: C:TCGAGCAGCGGCT(SEQ ID N0:70)
   - Primer pair:: Forward: CCGCTTCGCTCTCTTTGC(SEQ ID NO:71)
   Reverse: CCTCTGCTTCTTTGAGCTTGGA(SEQ ID NO:72)
For 3435 typing
   - Probe for detecting: C:CTCACGATCTCTTC(SEQ ID N0:73)
   - Probe for detecting: T:CCTCACAATCTCTT(SEQ ID NO:74)
   - Primer pair:: Forward: AACAGCCGGGTGGTGTCA(SEQ ID N0:75)
   Reverse: ATGTATGTTGGCCTCCTTTGCT(SEQ ID NO:76)

### Example 15

### (Association between the haplotypes and basal promoter activity of reporter constructs)

In order to examine the direct association between the haplotypes and MDR1 promoter activity, the 5' regulatory region between -2604 and -570 of genomic DNA from volunteers carrying three naturally occurring haplotypes (haplotypes 1, 2 and 3) was cloned. Then, the fragments to the reporter gene were ligated in the pGL3 basic vector. Because of the low frequencies of the polymorphisms at -1717 and -1325, genomic DNA with T monomorphic at -1717 and with A monomorphic at -1325 were used for reporter plasmid construction. These three constructs were then subjected to transient transfection in a human hepatoma cell line, HepG2. The promoter activity was analyzed after 48h of transfection and normalized with the co-transfected phRL-TK activity. The relative luciferase activity is shown by a rate when the activity of haplotype 1 construct is set to 100%. Data are shown as mean value ± S.D. (Stardard Deviation) of the associated expression for each of the 4 individual experiments. Each experiment was estimated by using 3 dishes (* P<0.05). As shown in Table 4, the minor-type construct carrying haplotypes 2 and 3 showed expression of 85.3±4.65% and 87.1±1.64%, respectively, of the major-type construct carrying haplotype 1. Together these experiments suggest that polymorphisms at the 5' regulatory region affect the basal promoter activity of reporter constructs containing the human MDR1 gene upstream promoter region.

**(Table 5) Basal promoter activity of reporter constructs containing -2604 to -570 of the human MDR1 gene harboring each haplotype**

| haplot ype | -241 0 | -235 2 | -191 0 | -93 4 | -69 2 | Luciferase activity (%) |
|---|---|---|---|---|---|---|
| 1 | T | G | T | A | T | 100 |
| 2 | T | A | T | A | T | 85.3 ± 4.65* |
| 3 | C | G | C | G | C | 87.1 ± 1.64* |

Relative luciferase activities are given as percentages of the activity of the haplotype1 construct, which was considered 100%. The data are expressed as means ± S.D. of relative expression in four independent experiments. Each experiment was assayed using triplicate dishes. *P<0.05

### Example 16

### (Identification of proteins differentially binding to major and minor alleles)

Electrophoretic mobility shift assays were used to investigate whether or not the SNPs of MDR1 5' regulatory region altered binding of nuclear proteins. We first examined -2352G>A (Figure 4A). A retarded band was observed when the probe -2352G was incubated with nuclear extracts of liver cells. This band was three times weaker than when -2352A was incubated. The specificity of the DNA-protein interaction was demonstrated by appropriate competition assays, i.e., the upper band almost completely disappeared under a 10-fold excess of the unlabeled oligonucleotide -2352G, while the addition of excess amounts of minor-type oligonucleotide -2352A did not inhibit the protein from binding to probe -2352G. Then, -692T>C was examined (Figure 4B). The allele-specific appearance of retarded band was also observed when the probe -692T was incubated with nuclear extracts. In competition assays, the upper band almost completely disappeared under a 10-fold excess of the unlabeled oligonucleotide -692T, and much weaker (nine times compared to -692T) inhibition of the binding was observed with the competitor -692C. Other oligonucleotide probes (-2410T>C, -1910T>C and -934A>G) showed no difference of protein-binding property between the major and minor types under the present conditions.

### Example 17

Similarly to MDR1 gene, SNPs for human MRP2 gene encoding ABC transporter were examined. Genomic DNA was extracted from bone marrow comprising leukocytes collected from infant leukemia patients with their informed consent, and polymorphism were detected by direct sequencing method for all of 32 exons of MRP2 gene and 4kb-upstream of promoter region, to identify 21 SNPs. The results are shown in Table 5. As for MDR 1 gene, probes for detecting each SNP, and primer sets are as follows:
For -3925 typing
   - Probe for detecting G:: CTGGTTGTAGGGCTTT (SEQ ID NO: 77)
   - Probe for detecting: A:CCTGGTTATAGGGCTTT(SEQ ID NO: 78)
   - Primer pair:: Forward: CGGGCTTCATTCAGAATTTTTTATCTTTGATT(SEQ ID NO: 79)
   Reverse: CACCAAGTAGAACAAATGCCAAACA(SEQ ID NO: 80)
For 3972 typing
   - Probe for detecting C:: ATGCTACCGATGTCAC(SEQ ID N0:81)
   - Probe for detecting T:: ATGCTACCAATGTCAC(SEQ ID N0:82)
   - Primer pair:: Forward: TGGTCCTCAGAGGGATCACTT(SEQ ID NO: 83)
   Reverse: TCCTTCACTCCACCTACCTTCTC(SEQ ID NO:84)
For -3933 typing
   - Probe for detecting C:: AAGTAAGGTCTCTTTCC(SEQ ID NO:85)
   - Probe for detecting T:: AAGTAAGGTCTTTTTCC(SEQ ID NO:86)
   - Primer pair :: Forward: GCTTGCTGAGGAAAAGTTGGACATA(SEQ ID NO:87)
   Reverse: AGTTGCAGGAAATCAAAGATAAAAAATTCTGAA(SEQ ID NO:88)
For 2366 typing
   - Probe for detecting: C:CATCCACTGCAGACAG(SEQ ID N0:89)
   - Probe for detecting: T:TCCACTGCAAACAG(SEQ ID N0:90)
   - Primer pair :: Forward: GCCAGAGCTACCTACCAAAATTTAGA(SEQ ID NO: 91)
   Reverse: GCCTTTCAACAGGCCATTGG(SEQ ID NO:92)
For -924 typing
   - Probe for detecting G:: AGGCCAAGGCAGAAG(SEQ ID N0:93)
   - Probe for detecting A:: AGGCCAAGACAGAAG(SEQ ID NO:94)
   - Primer pair:: Forward: GCAATCCCAGCCCTTTGG(SEQ ID N0:95)
   Reverse: CTCAAACTCCAGGCTTCAACAATC(SEQ ID N0:96)
For 1249 typing
   - Probe for detecting G:: CTGTTTCTCCAACGGTGTA(SEQ ID NO:97)
   - Probe for detecting A:: ACTGTTTCTCCAATGGTGTA(SEQ ID N0:98)
   - Primer pair:: Forward: CCAACTTGGCCAGGAAGGA(SEQ ID NO:99)
   Reverse: GGCATCCACAGACATCAGGTT(SEQ ID NO:100)
For typing intron 19
   - Probe for detecting C:: TCAAAGGAGAAGTGGTTTA(SEQ ID NO:101)
   - Probe for detecting T :: TTCAAAGGAGAAATGGTTTA(SEQ ID NO:102)
   - Primer pair:: Forward: CTGGATCTGAGTTTCTGGATTCTGT(SEQ ID NO:103)
   Reverse: GGGATGTTTTGCAAACTGTTCTTTG(SEQ ID NO:104)

**(Table 6) MRP2 genetic polymorphism of genes**

| | | | Genotype | | | Allele frequency | |
|---|---|---|---|---|---|---|---|
| Location | Nucleic acid Substitution | Amino acid Substitution | Maj/Maj | Maj/Min | Min/Min | Major | Minor |
| exon 10 | G1249A | Va1417IIe | 14 | 2 | 0 | 0.94 | 0.06 |
| exon 18 | C2366T | Ser789Phe | 15 | 1 | 0 | 0.97 | 0.03 |
| exon 22 | G2934A | Ser978Ser | 15 | 1 | 0 | 0.97 | 0.03 |
| exon 28 | C3972T | IIe1324IIe | 11 | 5 | 0 | 0.84 | 0.16 |
| 5' flanking | C-24T | - | 11 | 5 | 0 | 0.84 | 0.16 |
| promoter | A-920G | - | 11 | 5 | 0 | 0.84 | 0.16 |
| promoter | G-924A | - | 2 | 12 | 2 | 0.50 | 0.50 |
| promoter | G-1450A | - | 11 | 5 | 0 | 0.84 | 0.16 |
| promoter | G-1675T | - | 3 | 11 | 2 | 0.53 | 0.47 |
| promoter | A-1847C | - | 15 | 1 | 0 | 0.97 | 0.03 |
| promoter | C-3133G | - | 11 | 5 | 0 | 0.84 | 0.16 |
| promoter | A-3414T | - | 9 | 6 | 0 | 0.80 | 0.20 |
| promoter | A-3459C | - | 10 | 5 | 0 | 0.83 | 0.17 |
| promoter | G-3925A | - | 3 | 1 | 0 | 0.53 | 0.47 |
| promoter | T-3993C | - | 10 | 6 | 0 | 0.81 | 0.19 |
| Intron6 | T632+86A | - | 12 | 2 | 1 | 0.88 | 0.13 |
| Intron 14 | G1901-61A | - | 15 | 1 | 0 | 0.97 | 0.03 |
| Intron 19 | C2620-2133T | - | 8 | 7 | 1 | 0.72 | 0.28 |
| Intron 23 | C3258+56T | - | 11 | 5 | 0 | 0.84 | 0.16 |
| Intron 29 | G4146-584A | - | 14 | 1 | 1 | 0.91 | 0.09 |
| intron | C5199+317T | - | 11 | 5 | 0 | 0.84 | 0.16 |

### Industrial Applicability

According to the present invention, it is possible to determine haplotypes or diplotypes of MDR1 gene targeting the 5' regulatory region of MDR1 gene, being expressed in the apical membrane side and being an ABC transporter transporting a wide range of substrates, and by using the determination results of haplotypes or diplotypes of the 5'regulatory region of MDR1 gene of each individual as a marker of drug responsiveness, as well as the fundamental knowledge concerning SNPs of the 5'regulatory region of MDR1 gene, it is possible to perform tailor made treatment. Furthermore, as it is useful as a marker for estimating an oncogenic risk and its development, it is possible to develop it to a tailor made prevention by estimating the risk of cancer.

## Claims

1. A method for determining haplotypes and/or diplotypes of a 5'regulatory region of MDR1 gene, by detecting a polymorphism at a position selected from -2903, -2410, -2352, -1910, -1717 and -1325, when the position is indicated in relation to a first base of translation start codon (ATG) which is set to +1, in a base sequence of a 5'regulatory region of a MDR1 gene.

2. A method for determining haplotypes and/or diplotypes of a 5'regulatory region of MDR1 gene, by detecting polymorphism at a position selected from -934 and/or -692 position, in addition to the polymorphism at a position selected from -2903, -2410, -2352, -1910, -1717 and -1325, when the position is indicated in relation to a first base of translation start codon (ATG) which is set to +1, in a base sequence of a 5'regulatory region of a MDR1 gene.

3. The method for determining haplotypes and/or diplotypes of a 5'regulatory region of MDR1 gene according to claim 1 or 2, comprising the step of investigating whether the base at -2903 is thymine or cytosine.

4. The method for determining haplotypes and/or diplotypes of a 5'regulatory region of MDR1 gene according to any one of claims 1 to 3, comprising the step of investigating whether the base at -2410 is thymine or cytosine.

5. The method for determining haplotypes and/or diplotypes of a 5'regulatory region of MDR1 gene according to any one of claims 1 to 4, comprising the step of investigating whether the base at -2352 is guanine or adenine.

6. The method for determining haplotypes and/or diplotypes of a 5'regulatory region of MDR1 gene according to any one of claims 1 to 5, comprising the step of investigating whether the base at -1910 is thymine or cytosine.

7. The method for determining haplotypes and/or diplotypes of a 5'regulatory region of MDR1 gene according to any one of claims 1 to 6, comprising the step of investigating whether the base at -1717 is thymine or cytosine.

8. The method for determining haplotypes and/or diplotypes of a 5'regulatory region of MDR1 gene according to any one of claims 1 to 7, comprising the step of investigating whether the base at -1325 is guanine or adenine.

9. A DNA of 5' regulatory region of MDR1 gene, wherein bases at -2410, -2352, -1910, -934, -692 are replaced with thymine, adenine, thymine, adenine, thymine, respectively, when the position is indicated in relation to a first base of translation start codon (ATG) which is set to +1, in a base sequence of a 5'regulatory region of MDR1 gene.

10. A DNA of 5' regulatory region of MDR1 gene, wherein bases at -2410, -2352, -1910, -934, -692 are replaced with cytosine, guanine, cytosine, guanine, cytosine, respectively, when the position is indicated in relation to a first base of translation start codon (ATG) which is set to +1, in a base sequence of a 5'regulatory region of MDR1 gene.

11. A DNA of 5' regulatory region of MDR1 gene, wherein bases at -2410, -2352, -1910, -934, -692 are replaced with cytosine, adenine, cytosine, guanine, cytosine, respectively, when the position is indicated in relation to a first base of translation start codon (ATG) which is set to +1, in a base sequence of a 5'regulatory region of MDR1 gene.

12. A DNA of 5' regulatory region of MDR1 gene, wherein bases at -2410, -2352, -1910, -934, -692 are replaced with thymine, adenine, thymine, guanine, thymine, respectively, when the position is indicated in relation to a first base of translation start codon (ATG) which is set to +1, in a base sequence of a 5'regulatory region of MDR1 gene.

13. A primer set comprising a forward primer that hybridizes with a region upstream of a position for detecting polymorphism, and a reverse primer that hybridizes with a region downstream of a position for detecting polymorphism, which is used for a method for determining haplotypes of a 5' regulatory region of MDR1 gene for detecting a polymorphism at a position selected from -2903, -2410, -2352, -1910, -1717 and -1325, when the position is indicated in relation to a first base of translation start codon (ATG) which is set to +1, in a base sequence of a 5'regulatory region of MDR1 gene.

14. A method for determining diplotype of 5' regulatory region of MDR1 gene by detecting a polymorphism at -2352, and at a position selected from -2410, -1910 and -692, when the position is indicated in relation to a first base of translation start codon (ATG) which is set to +1, in a base sequence of a 5'regulatory region of MDR1 gene.

15. The method for determining diplotype of 5' regulatory region of MDR 1 gene according to claim 14, wherein gene-typing is performed by TaqMan (registered trademark) method.

16. A probe and a primer set, used for a method for determining diplotype of 5' regulatory region of MDR1 gene by detecting a polymorphism at -2352, and at a position selected from -2410, -1910 and -692, when the position is indicated in relation to a first base of translation start codon (ATG) which is set to +1, in a base sequence of a 5'regulatory region of MDR1 gene.

17. The probe and the primer set according to claim 16, used for a method for determining diplotype of 5' regulatory region of MDR1 gene by TaqMan (registered trademark) method.

18. A method for estimating an onset of colon cancer, wherein the method for determining haplotypes and/or diplotypes of 5' regulatory region of MDR1 gene according to any one of claims 1 to 8, or the method for determining diplotype of 5'regulatory region of MDR1 gene according to claim 14 or 15 is used.

19. A method for developing a drug for controlling MDR1 expression, wherein at least one position selected from -2903, -2410, -2352, -1910, -1717 and -1325, -934, -692 is targeted, when the position is indicated in relation to a first base of translation start codon (ATG) which is set to +1, in a base sequence of a 5'regulatory region of MDR1 gene.
